# DEMANDE DE BREVET EUROPEEN

(11) **EP 4 850 632 A1**
(43) Date de publication de la demande: **09.12.2026**
(21) Numéro de dépôt: 26183145.7
(22) Date de dépôt: 04.06.2026
(51) Int. Cl.: A61C 8/02, A61B 17/80, A61F 2/28

(54) **NOUVEAU DISPOSITIF DE REGENERATION OSSEUSE**

(30) Priorité: 06.06.2025 FR 2506176
(71) Demandeur: BIOTECH DENTAL, 13300 Salon-de-Provence (FR)
(72) Inventeur: KELLER, Pierre, 67000 Strasbourg (FR)
(74) Mandataire: Hautier IP

(57) **Abrégé**

Dispositif destiné à la régénération osseuse d'un défaut au sein d'un os de la mâchoire, comprenant au moins une plaque de recouvrement dont la forme recrée la surface de l'os telle qu'elle était avant l'apparition du défaut et qui recouvre ce défaut tout en reposant en partie sur l'os sain environnant. La plaque de recouvrement présente des micro-perforations et intègre au moins une ligne de rupture prédéfinie s'étendant de sa périphérie vers son centre, dont la longueur et l'orientation permettent de guider une ouverture selon un tracé défini sur au moins 25 % de sa largeur.

## Description

### DOMAINE DE L'INVENTION

La présente invention est relative au domaine des dispositifs destinés à la régénération osseuse.

### ART ANTERIEUR

Une destruction du tissu osseux peut parfois s'opérer avec une origine qui peut être infectieuse, tumorale, ou traumatique. Cette destruction est normalement suivie d'une cicatrisation osseuse. Cependant, dans certains cas et notamment avec le vieillissement, cette cicatrisation osseuse peut s'avérer insuffisante pour régénérer l'intégrité du tissu détruit entrainant l'apparition de défauts osseux. Ces défauts osseux correspondent alors à des cavités en lieu et place de l'os détruit. De tels défauts osseux peuvent s'avérer particulièrement problématiques dans le domaine dentaire, dans le cadre de la pose d'un implant dentaire.

Un implant dentaire est une vis servant de racine artificielle visant à la mise en place d'une prothèse dentaire qui vient s'implanter dans un os de la mâchoire. En fait de « mâchoire », on parle des mâchoires qui sont les deux masses osseuses opposées délimitant la cavité buccale. La mâchoire inférieure, appelée mandibule, os mandibulaire ou encore maxillaire inférieur, constitue la portion mobile, tandis que la mâchoire supérieure, appelée maxillaire, os maxillaire ou encore maxillaire supérieur en opposition avec le maxillaire inférieur correspond à la portion mobile. Chaque mâchoire, inférieure ou supérieur, possède une arcade alvéolaire, structure osseuse en forme de fer à cheval contenant les alvéoles dentaires, cavités recevant les racines des dents. Cette arcade alvéolaire présente deux côtés distincts, le côté interne et le côté externe. Le côté interne de l'os, vers l'intérieur de la bouche, est dit lingual pour la mandibule, face à la langue, et palatin pour le maxillaire, face au palais. Le côté externe de l'os, orienté vers l'extérieur de la bouche, est dénommé vestibulaire pour la mandibule ou le maxillaire, car il fait face aux lèvres et aux joues formant le vestibule buccal. Cette arcade alvéolaire présente une crête, suivant la ligne d'implantation des dents et de leurs alvéoles qui définit un arc caractéristique, l'arc alvéolaire. Compte tenu de ces données anatomiques et pour présenter une résistance mécanique suffisante, un implant dentaire va présenter généralement une longueur d'au moins 8 mm et un diamètre supérieur ou égal à 3,5 mm. Une telle dimension d'implant implique que l'os de la mâchoire, dans lequel est positionné l'implant, ait des dimensions suffisantes pour parfaitement enrober cet implant. Maintenant, et du fait d'un défaut osseux au niveau de la mandibule, maxillaire inférieur, ou de l'os maxillaire, maxillaire supérieur, il est courant que la pose d'un tel implant ne soit alors pas possible à moins de procéder à une régénération osseuse préalable au niveau de ce défaut.

Face à de tels défauts osseux de la mâchoire, on procède à une régénération osseuse qui utilise des biomatériaux permettant de les combler et de favoriser la cicatrisation osseuse en leur sein. En règle générale, le biomatériau utilisé est constitué soit de matériau de substitution osseuse synthétique (par exemple des granules d'hydroxyapatite), soit de particules d'os. Une fois l'évidement correspondant au défaut osseux rempli par ce biomatériau, cet évidement est fermé par une pièce de recouvrement qui permet de diriger la croissance de ce biomatériau. Cette pièce de recouvrement est fixée à l'os entourant le défaut osseux en vue d'encadrer et de protéger le volume destiné à la régénération osseuse. Une telle technique, surtout en lien avec l'os de la mâchoire dont les caractéristiques et l'accessibilité peuvent s'avérer problématiques, nécessite alors un maximum d'habileté manuelle de la part du chirurgien. Après quelques mois et une fois la régénération osseuse opérée, le chirurgien pourra poursuivre les opérations nécessaires sur le patient en retirant, si nécessaire, la pièce de recouvrement alors inutile avant de procéder à la pose d'un ou plusieurs implants.

Sur cette base, différentes pièces de recouvrement ont pu être développées par le passé.

Un premier type de pièces de recouvrement, prenant la forme de membranes (souple, ex : membrane type PRF) a été développé. Ces membranes sont positionnées, par le praticien, sur le substitut osseux après remplissage du défaut osseux, avant d'être fixées par une ou plusieurs vis à l'os sain adjacent au défaut osseux. A titre d'exemple de tels membranes, on peut citer le brevet US 4,816,339 qui décrit une membrane de recouvrement synthétique et multicouches.

Un second type de pièces de recouvrement, prenant la forme de plaques (rigides, Ex : membrane décrite dans le brevet EP 2 536 446 B1) a ensuite été développé. Si les plaques de recouvrement permettent, par rapport aux membranes de recouvrement, un ajustement bien plus précis sur l'os et un processus de régénération bien plus encadré (du fait de la meilleure délimitation de la régénération et de la meilleure stabilité par exemple suite à la mastication), elles présentent une complexité de mise en oeuvre et de retrait bien plus importante, limitant leurs utilisations à des chirurgiens bénéficiant d'un niveau de pratique et d'expertise élevé. En effet, leur rigidité va nécessiter une préparation parfaite du site de pose de même qu'une parfaite réalisation de cette pose (ouverture de l'os de la mâchoire, préparation et nettoyage du site de défaut osseux, pose homogène et adapté du substitut de régénération osseuse au sein du site de régénération osseuse jusqu'à obtenir le volume de régénération osseuse souhaité sur l'os, pose de la plaque de recouvrement rigide enveloppant ledit volume de régénération et reposant simultanément sur de l'os sain encadrant le défaut osseux, fixation de la plaque de recouvrement sur l'os sain (et donc sur la face de l'os de la mâchoire tournée vers l'intérieur de la bouche du patient, fermeture des tissus mous sur la plaque de recouvrement pour permettre la régénération osseuse). De même, les caractéristiques de ces plaques de recouvrement vont nécessiter une dépose tout aussi complexe. En effet, même dirigée, la régénération osseuse forme souvent des excroissances (de type boursouflures) à son extrémité. La plaque de recouvrement, dont la surface est parfaitement associée au volume de régénération se trouve souvent légèrement déformée au niveau de ces boursouflures de sorte que son retrait peut entraîner l'arrachage du tissu régénéré, surtout au niveau de ces excroissances.

Face à cette double-problématique de pose et de retrait, le brevet EP 3 294 222 B1 décrit une plaque de recouvrement rigide et largement ajourée qui intègre simultanément des moyens de positionnement, pour en faciliter la pose, et au moins un point de rupture prédéterminé pour en faciliter le retrait. Maintenant, une telle plaque de recouvrement, du fait de sa structure largement ajourée, également au niveau de son point de rupture prédéterminé, se retrouve largement colonisée lors de la régénération osseuse, ce qui complexifie son retrait, quand bien même le praticien utiliserait son point de rupture prédéterminé.

Il en résulte que les praticiens sont toujours demandeurs aujourd'hui de plaques de recouvrement qui permettrait simultanément une pose et un retrait facilité.

### RESUME DE L'INVENTION

Face à cette problématique, les inventeurs ont développé une plaque de recouvrement qui présente dont la structure microperforée lui confère tout à la fois une certaine malléabilité tout en empêchant sa colonisation lors de la régénération osseuse, et qui présente en outre au moins une ligne de rupture prédéfinie qui s'étend du pourtour de la plaque de recouvrement vers son axe longitudinal de sorte à réduire les risques de dommages du tissu, notamment d'arrachage du tissu osseux régénéré, et ainsi faciliter largement le travail de pose et de retrait du praticien tout en améliorant le confort du patient.

Aussi, un premier objet de l'invention se rapporte à un dispositif (1) destiné à la régénération osseuse d'un défaut osseux au sein d'un os de la mâchoire et qui comprend :
i) au moins une plaque de recouvrement dont :
   - la forme permet de recréer la surface de l'os de la mâchoire, préalablement à l'apparition du défaut osseux et en recouvrant ledit défaut osseux, tout en s'appuyant, au moins partiellement, sur l'os sain encadrant ledit défaut osseux,
   - la surface, recouvrant ledit défaut osseux, présente des micro-perforations (4), laquelle consiste typiquement en une surface micro-perforée, et
ii) au moins un moyen de fixation,
où la plaque de recouvrement comprend, en outre, au moins une ligne de rupture prédéfinie qui :
- s'étend, transversalement à l'axe longitudinal de la plaque de recouvrement, de la périphérie de la plaque de recouvrement vers son centre, et
- présente une longueur et une orientation permettant de diriger la rupture de la plaque de recouvrement selon un tracé prédéterminé de sorte à opérer une ouverture contrôlée de cette plaque de recouvrement sur au moins 25% de sa largeur.

En outre, les inventeurs ont mis en évidence qu'un positionnement lingual ou palatin de cette ligne de rupture prédéfinie facilite grandement le retrait de la plaque de recouvrement par le praticien, surtout lorsque la plaque de recouvrement ne présente en outre aucun orifice de fixation du côté de la cavité linguale ou palatine.

Selon un mode de réalisation préféré, ladite au moins une ligne de rupture prédéfinie est disposée, par rapport à l'axe longitudinal de la plaque de recouvrement, sur la surface de la plaque de recouvrement venant en appui sur le côté interne de l'os (vers l'intérieur de la bouche). Comme mentionné précédemment, ce côté interne de l'os est qualifié de face linguale pour la mandibule et de face palatine pour le maxillaire.

Avantageusement, ladite au moins une plaque de recouvrement ne comprend aucun orifice de passage positionné sur la surface de la plaque de recouvrement venant en appui sur le côté interne de l'os (vers l'intérieur de la bouche).

Les inventeurs ont également mis en évidence qu'un élargissement de la ligne de rupture au niveau d'une de ses extrémités, permet d'arrêter précisément l'ouverture de la ligne de rupture prédéfinie au niveau de cet élargissement, facilitant ainsi le contrôle de la rupture par le praticien et limitant les risques pour le patient.

Selon un autre mode de réalisation préféré, l'une au moins des extrémités de ladite au moins une ligne de rupture prédéfinie comprend un élargissement de celle-ci, lequel élargissement prend de préférence la forme d'un cercle.

Maintenant, les inventeurs ont mis en évidence qu'un tel élargissement, s'il est positionné au niveau de l'extrémité la plus au centre de la plaque de recouvrement, ou extrémité non-périphérique (en opposition à son extrémité positionnée à la périphérie de la plaque de recouvrement), d'une ligne de rupture prédéfinie peut s'avérer particulièrement intéressant, lorsque cette extrémité interne est positionnée précisément au niveau du point de l'os de la mâchoire où, après retrait de la plaque de recouvrement, le praticien doit positionner un implant. En effet, une telle plaque de recouvrement permet alors simplement et rapidement au praticien de pratiquer une ouverture, au sein de la plaque de recouvrement et au niveau de cette extrémité interne d'une ligne de rupture prédéfinie, par exemple par perçage, de sorte à étendre ensuite cette ouverture de la plaque de recouvrement à la ligne de rupture prédéfinie partant de cette extrémité interne, sans avoir à craindre de détériorer l'os régénéré sous-jacent sachant qu'il sera foré à ce niveau par la suite pour recevoir un implant.

Selon un mode de réalisation préféré complémentaire, l'extrémité de l'au moins une ligne de rupture prédéfinie comprenant un élargissement de celle-ci est l'extrémité non-périphérique de cette au moins une ligne de rupture, laquelle extrémité se positionne, sur la plaque de recouvrement, au niveau d'un futur site d'implantation dans l'os de la mâchoire après régénération osseuse et retrait de la plaque de recouvrement.

De préférence, cette extrémité présente une forme distincte de celle de la ligne de rupture prédéfinie. Ainsi, elle pourra constituer un repère visuel clair, permettant d'aider le praticien dans l'identification et l'utilisation de la ligne de rupture associée concernée.

Les inventeurs ont également mis en évidence qu'un rétrécissement de la surface périphérique dans le prolongement de ladite au moins une ligne de rupture prédéfinie facilite grandement l'ouverture de la ligne de rupture prédéfinie sans affecter la qualité de la mise en place de la plaque de recouvrement.

Selon un mode de réalisation préféré, l'extrémité « périphérique » de l'au moins une ligne de rupture est positionnée au sein de cette bande ou surface périphérique de la plaque de recouvrement ou à sa proximité immédiate, laquelle surface périphérique constitue en outre une bande discontinue dont la largeur est plus faible, voire nulle, dans le prolongement de cette au moins une ligne de rupture prédéfinie.

Les inventeurs ont enfin mis en évidence que l'intégration d'un moyen de préhension, à destination du praticien, à proximité immédiate de ce rétrécissement de la bordure permet au praticien de procéder à l'ouverture selon la ligne de rupture prédéfinie avec une bien plus grande facilité.

Aussi, et selon un mode de réalisation préféré complémentaire, la surface périphérique comprend au moins un moyen de préhension sur au moins un des 2 côtés encadrant le rétrécissement venant dans le prolongement de ladite au moins une ligne de rupture prédéfinie, lequel moyen de préhension prend de préférence la forme d'un élargissement de la bande discontinue sur au moins un des 2 côtés encadrant ce rétrécissement venant dans le prolongement de ladite au moins une ligne de rupture prédéfinie, lequel élargissement prenant typiquement la forme une languette.

Enfin, un deuxième objet de l'invention porte sur une plaque de recouvrement telle que définie précédemment.

### DESCRIPTION DES FIGURES

La figure 1 est une représentation d'un mode de réalisation d'une plaque de recouvrement d'un dispositif selon l'invention.
La figure 2 est une représentation d'un mode de réalisation d'une plaque de recouvrement d'un dispositif comprenant deux élargissements non périphériques présentant un fond conique.
La figure 3 est une représentation d'un mode de réalisation d'une plaque de recouvrement d'un dispositif selon l'invention qui comprend des élargissements périphériques au niveau de la surface périphérique.
La figure 4 est une représentation d'un mode de réalisation d'une plaque de recouvrement d'un dispositif selon l'invention qui présente des moyens de préhension en forme de languette.

### DESCRIPTION DETAILLEE DE L'INVENTION

La plaque de recouvrement peut présenter une forme arrondie, rectangulaire ou, plus généralement, toute forme adaptée à la surface de l'os de la mâchoire de destination. Maintenant, et dans le cas d'une plaque de recouvrement de forme rectangulaire, on prendra soin d'en arrondir les angles en vue de ne pas risquer de blesser le périoste ou les tissus mous.

Les dimensions de cette plaque de recouvrement sont telles qu'elle recouvre intégralement le défaut osseux avec, en outre, au moins 5 % de sa surface s'appuyant sur l'os sain encadrant ledit défaut osseux, de préférence au moins 10 % de sa surface.

Typiquement, le défaut osseux présente :
- une longueur comprise entre 5 et 200 mm, de préférence entre 10 et 100 mm ;
- une largeur comprise entre 3 et 20 mm, de préférence entre 5 et 10 mm ; et
- une profondeur comprise entre 5 et 20 mm, de préférence entre 8 et 15 mm.

Le plus généralement, le défaut osseux s'inscrit dans l'arc alvéolaire et la plaque de recouvrement se positionnera donc sur l'arc alvéolaire.

Aussi, et dans ce cas, l'axe longitudinal de la plaque de recouvrement se superpose avec l'arc alvéolaire.

Les micro-perforations de la surface microperforée de la plaque de recouvrement permettent, en les laissant traverser l'épaisseur de la plaque, l'apport d'éléments nutritifs à l'os en régénération sur le défaut osseux. De préférence, la répartition des micro-perforations est homogène au sein de la surface de la plaque recouvrant le défaut osseux. Maintenant, la taille de ces micro-perforations empêche le passage des cellules et donc la colonisation de la plaque de recouvrement par des tissus (mous et/ou durs), facilitant d'autant la dépose de la plaque de recouvrement si cela était nécessaire. Typiquement, on entend par micro-perforations, des perforations qui présentent un diamètre compris entre 10 et 500 µm, de préférence entre 50 et 100µm.

Avantageusement, la densité des micro-perforations est telle qu'elle permet une régénération osseuse optimale tout en contribuant à l'élasticité de la plaque de recouvrement de sorte à en faciliter sa pose par le praticien. En outre, la part de la surface occupée par les micro-perforations, au regard de la surface de la plaque de recouvrement recouvrant ledit défaut osseux, est telle qu'elle permet un apport maximal d'éléments nutritifs vers le défaut osseux et donc sa régénération optimale.

A ce titre, la densité de micro-perforations au sein de la surface de la plaque recouvrant le défaut osseux est comprise entre 10 à 500 perforations/cm², de préférence entre 20 à 250 perforations/cm² et, de façon particulièrement préférée, entre 50 à 200 perforations/cm².

A considérer la surface occupée par les micro-perforations, celle-ci correspond à au moins 10% de la surface totale de la plaque recouvrant ledit défaut osseux, et de façon particulièrement préférée, au moins 15%. Maintenant, la surface occupée par les micro-perforations est de préférence inférieure à 50% de la surface totale de la plaque recouvrant le défaut osseux, et de façon particulièrement préférée, inférieure ou égale à 25% de cette surface totale.

Pour améliorer la régénération osseuse, les micro-perforations pourront être associées à au moins un facteur de croissance sur leur surface interne. A titre d'exemple de tels facteurs de croissance, on peut citer le « Bone Morphogenetic Protein-2 » (BMP-2).

A considérer toujours les micro-perforations, la périphérie de la plaque de recouvrement en est préférentiellement dépourvue pour en améliorer l'appui.

Avantageusement, la surface périphérique de la plaque de recouvrement s'appuyant sur l'os sain, et encadrant ledit défaut osseux, est dépourvue de micro-perforations.

Une telle surface périphérique dépourvue de micro-perforation constituera une bande qui forme tout ou partie du pourtour de la plaque de recouvrement dont la largeur est comprise entre 0,5 et 5 mm, de préférence entre 1 et 2 mm.

On entend par « ligne de rupture prédéfinie », appelée aussi parfois « ligne frangible », une ligne au sein de la plaque de recouvrement qui est structuralement prédisposée à la rupture et adaptée pour concentrer les contraintes mécaniques de sorte que, en cas de charge de traction appliquée sur celle-ci ou à proximité, une rupture s'opère au sein de la plaque de recouvrement selon cette ligne. Du fait de cette au moins une ligne de rupture prédéfinie, il est possible au praticien de libérer largement la zone osseuse régénérée sans pour autant fragmenter la plaque de recouvrement, de sorte à faciliter son retrait, sans endommager ni la zone osseuse régénérée, ni les tissus adjacents.

Ladite au moins une ligne de rupture prédéfinie est une ligne droite ou une ligne courbe. De préférence, ladite au moins une ligne de rupture prédéfinie est une ligne droite (rectiligne).

Si ladite au moins une ligne de rupture prédéfinie coupe de préférence perpendiculairement l'axe longitudinal de la plaque de recouvrement, elle peut couper tout aussi bien cet axe longitudinal selon une orientation distincte (c'est-à-dire un angle compris entre 10 et 90°).

Avantageusement, la longueur et l'orientation de ladite au moins une ligne de rupture prédéfinie permettent ensemble de diriger la rupture de la plaque de recouvrement selon le tracé prédéterminé par cette une ligne et d'opérer une ouverture contrôlée de cette plaque de recouvrement sur 25% à 75% de sa largeur, de préférence sur 30 à 60% et, de manière particulièrement préférée, sur 35 à 55% de sa largeur.

Ainsi, dans le cas d'une ligne de rupture prédéfinie prenant la forme d'une ligne droite qui coupe perpendiculairement l'axe longitudinal de la plaque de recouvrement, cette ligne de rupture prédéfinie présentera ainsi une longueur allant de 25% à 75% de la largeur de la plaque de recouvrement, de préférence de 30 à 60% et, de manière particulièrement préférée, de 35 à 55% de la largeur de la plaque de recouvrement.

Selon un mode de réalisation préféré, ladite au moins une ligne de rupture prédéfinie est disposée, par rapport à l'axe longitudinal de la plaque de recouvrement, sur la surface de la plaque de recouvrement venant en appui sur le côté interne de l'os (vers l'intérieur de la bouche).

La largeur de la ligne de rupture prédéfinie variera en fonction de sa nature, mais sera généralement inférieure à 5 mm, de préférence elle sera inférieure à 3 mm.

Avantageusement encore, la largeur de la ligne de rupture prédéfinie sera comprise entre 0,1 mm et 3 mm, de façon préférée, entre 0,3 mm et 2,5 mm et, de façon particulièrement préférée, entre 0,5 et 2 mm.

Alternativement, la largeur de la ligne de rupture prédéfinie est égale au diamètre des micro-perforations au sein de la plaque de recouvrement.

Typiquement, la plaque de recouvrement comprendra plusieurs lignes de rupture prédéfinies et d'autant plus qu'elle sera longue. Une telle configuration permet de désolidariser, autant que possible, la plaque de recouvrement de l'os régénéré, de sorte à faciliter son retrait et à limiter les risques de sa fragmentation.

Avantageusement, la plaque de recouvrement comprend au moins deux lignes de rupture prédéfinies et, de préférence, au moins trois lignes de rupture prédéfinies.

Typiquement, l'écartement entre deux lignes de rupture prédéfinies adjacentes est régulier au sein de la plaque de recouvrement (pour une plaque de recouvrement comptant plus de trois lignes de rupture prédéfinies adjacentes).

Avantageusement encore, la plaque de recouvrement comprend au moins une ligne de rupture prédéfinie par intervalle de longueur de plaque de recouvrement compris entre 10 et 20 millimètres.

En termes d'orientation, deux lignes de rupture prédéfinies adjacentes pourront partager ou non une même orientation.

Ainsi, et à titre d'exemple, deux lignes de rupture prédéfinies adjacentes, et constituant chaque une ligne droite, pourront être parallèles.

Alternativement, et toujours à titre d'exemple, deux lignes de rupture prédéfinies adjacentes, et constituant chaque une ligne droite, pourront avoir chacune une direction distincte et voir leurs deux extrémités internes à la plaque de recouvrement se rejoindre, de préférence au milieu de la largeur de la plaque de recouvrement ou à proximité.

Selon un autre mode de réalisation préféré, deux lignes de rupture prédéfinies adjacentes partagent une même orientation. Dans le cas de deux lignes de rupture prédéfinies adjacentes prenant la forme de lignes droites, ces deux lignes seront donc parallèles.

Une telle ligne de rupture prédéfinie peut être réalisée par des techniques bien connues de l'homme du métier. A titre d'exemple, une telle ligne de rupture prédéfinie pourra prendre la forme d'une ligne de moindre épaisseur sur la plaque de recouvrement, obtenue par exemple lors du moulage ou de l'impression 3D de la plaque ou lors d'un usinage. A titre d'exemple encore, une telle ligne de rupture prédéfinie pourra prendre la forme d'une ligne formée d'un matériau de résistance mécanique moindre au sein de la plaque de recouvrement (par rapport à celui utilisé pour le reste de la plaque de recouvrement et obtenue par exemple lors d'une impression 3D de la plaque de recouvrement à l'aide d'une imprimante à même d'utiliser plusieurs matériaux, ex : imprimante 3D avec tête multi-filaments).

Avantageusement toujours, ladite au moins une ligne de rupture prédéfinie pourra présenter une structure continue ou une structure discontinue.

Dans le cas d'une ligne de rupture prédéfinie correspondant à une ligne de moindre épaisseur, une telle structure discontinue s'apparentera à une ligne présentant des segments successifs d'épaisseurs distinctes. A noter que de tels segments, intercalés entre les deux extrémités de la ligne de rupture, pourront même aller jusqu'à avoir une épaisseur nulle (c'est-à-dire correspondre à un jour, d'ores et déjà à une ouverture).

Avant de définir plus avant cette ligne de rupture prédéfinie, il convient de définir plus précisément la plaque de recouvrement à laquelle elle est associée et ses caractéristiques.

Typiquement, la plaque de recouvrement sera réalisée dans un matériau métallique, rigide, biocompatible et non cassant.

Si une telle plaque de recouvrement peut être obtenue par toute méthode connue de l'homme du métier, que ce soit le moulage ou le orifice de passage, elle est préférentiellement fabriquée par impression 3D. Une telle impression 3D utilise typiquement une poudre métallique dont la mise en forme en plaque de recouvrement est assurée par un laser de puissance adaptée. Cette approche est particulièrement intéressante en ce qu'elle garantit une restauration anatomique fidèle, en s'appuyant sur des données historiques de l'anatomie du patient.

De préférence, la plaque de recouvrement est réalisée en titane ou en alliage de titane.

Pour présenter ses propriétés particulièrement avantageuses, la plaque de recouvrement présente une épaisseur comprise entre 0,1 et 1,5 mm, de préférence entre 0,3 et 1 mm et, de manière particulièrement préférée entre 0,4 et 0,9 mm.

A considérer le titane, il s'agit d'un métal de transition brillant avec une faible densité et une résistance élevée. Le titane « pur » est ainsi plus résistant que les aciers ordinaires à faible teneur en carbone, tout en étant 45 % plus léger.

Maintenant, le titane n'est jamais strictement pur et on trouve toujours en son sein des traces d'autres éléments (oxygène et d'autres composants). Aussi, il existe une classification spécifique pour le titane, on parle des « grades », laquelle est relative à sa pureté. Dans le détail, on parlera de titane « pur » pour les titanes de grade 1 à 4 et d'alliages de titane pour les titanes de grade 5 et au-delà. Ainsi, le titane le plus pur est le titane de grade 1, qui contient tout de même 0,18% d'oxygène, et le titane de grade 4 est le titane le moins pur (des 4 grades), avec une proportion de 0,4% d'oxygène. Pour les alliages et à titre d'exemple, le titane de grade 5 comprend 6% d'aluminium et 4% de vanadium, le titane de grade 12 comprend 3% de molybdène et 0,8% de nickel, et le titane de grade 38 avec 4% d'aluminium, 2,5% de vanadium et 1,5% de fer.

Outre leur composition spécifique, le titane solide et ses alliages peuvent se trouver sous la forme de deux structures cristallines, à savoir hexagonale compacte (phase α et au-dessous de 880 °C pour le titane « pur ») et/ou cubique centrée (phase β ; au-delà de 880 °C pour le titane « pur »). Ce dimorphisme est à la base d'une classification correspondant aux familles « *alpha* », « alpha-bêta » et « *bêta* » avec chacune des propriétés d'usage particulières. Ainsi, les plus hautes résistances mécaniques sont obtenues avec les alliages β alors que le titane « pur » appartient lui à la famille alpha. Maintenant, il est possible d'effectuer un traitement thermique sur le titane pur ou de l'un de ses alliages, on parle de « recuit ». Ce traitement correspond à une montée en température contrôlée du titane ou de l'un de ses alliages de sorte à entrainer une transition d'une structure cristalline en phase α vers une structure cristalline en phase β, la stabilité de cette transition pouvant varier.

Au final, les propriétés des différents grades de titane différent considérablement entre eux, notamment en ce qui concerne leur ductilité et leur résistance mécanique.

Il en résulte qu'on utilise majoritairement aujourd'hui le titane de grade 4 pour les plaques de recouvrement, du fait de ses meilleures propriétés mécaniques. A noter que le titane de grade 5 (alliage de titane avec du niobium), dont la dureté est supérieure au titane de grade 4, se substitue de plus en plus au titane de grade 4 pour la fabrication des composants prothétiques. A l'inverse, le titane de grade 2, utilisé par le passé, du fait d'une meilleure disponibilité, n'est presque plus utilisé en implantologie du fait de sa moindre résistance, alors que le titane de grade 1 n'a tout simplement jamais été véritablement utilisé du fait de sa malléabilité significative. Pour des revues sur l'utilisation actuelle du titane et de ses alliages en implantologie, on peut consulter LIU et al. (Regenerative biomaterials, vol.4, page 315 à 323, 2017) ou NICHOLSON (Prosthesis, vol.2, page 100 à 116, 2020).

Typiquement, la plaque de recouvrement pourra donc être réalisée en titane de grade 4 ou 5.

Maintenant, il a été mis en évidence qu'une plaque de recouvrement réalisée en titane de grade 1 ou 2 présente des propriétés de ductilité et de résistance mécanique particulièrement adaptée, de sorte qu'elle permet tout à la fois de bien contenir le volume de régénération osseuse sans souffrir de déformations, par exemple du fait de mastications inadaptées du patient, tout en bénéficiant d'une certaine malléabilité facilitant largement le travail de pose sur le défaut osseux du patient par le praticien.

A considérer les caractéristiques mécaniques de la plaque de recouvrement en titane de grade 1 ou de grade 2, elles ont permis du fait de leur rigidité et comme cela a pu être possible pour les plaques de recouvrement en titane de grade 4 ou de grade 5, de se passer de fixation sur l'os de la mâchoire du côté de la crête palatine (du côté de la cavité linguale) qui est difficile d'accès pour le praticien.

Aussi, et selon un mode de réalisation préféré, la plaque de recouvrement est réalisée en titane de grade 1 ou 2.

Pour revenir à la ligne de rupture prédéfinie et selon un autre mode de réalisation préféré, ladite ligne de rupture prédéfinie est une ligne de moindre épaisseur (potentiellement une rainure) au sein de la plaque de recouvrement.

Typiquement, cette ligne de rupture prédéfinie prendra la forme d'une ligne, au sein de la plaque de recouvrement, dont elle constitue une zone de moindre épaisseur, au niveau de laquelle l'épaisseur de la plaque de recouvrement est inférieure ou égale à 75% de l'épaisseur de la plaque de recouvrement de part et d'autre de celle-ci, de préférence inférieure ou égale à 50% et, de manière particulièrement préférée, inférieure ou égale à 33% de l'épaisseur de la plaque de recouvrement de part et d'autre de cette même ligne.

Cette ligne de rupture prédéfinie pourra se matérialiser par une moindre épaisseur sur l'une et/ou l'autre des deux faces de la plaque de recouvrement, à savoir la face externe et/ou la face interne de la plaque de recouvrement.

Par « face interne » de la plaque de recouvrement, on désigne sa face qui est en contact avec l'os de la mâchoire et avec le substitut de régénération osseuse.

Par « face externe » de la plaque de recouvrement, on désigne sa face opposée à sa face interne.

Avantageusement, la ligne de rupture prédéfinie se matérialisera par une moindre épaisseur sur la face externe de la plaque de recouvrement.

Ce positionnement est particulièrement adapté puisqu'il permet, au praticien, de visualiser au mieux la ligne de rupture prédéfinie de sorte à faciliter l'opération de retrait de la plaque de recouvrement.

De préférence, la ligne de rupture prédéfinie se matérialisera par une moindre épaisseur sur la face externe et la face interne de la plaque de recouvrement.

Ce positionnement est particulièrement adapté pour assurer une rupture sous charge optimale.

Dans le cas d'une structure discontinue, l'épaisseur de la ligne de rupture prédéfinie pourra être localement nulle sur certains segments.

De tels segments d'épaisseur nulle, ou segments ajourés, au sein de la ligne de rupture prédéfinie permettent d'affiner le contrôle de la rupture et de réduire la résistance mécanique de manière ciblée.

Ces segments d'épaisseur nulle pourront présenter une longueur et, potentiellement, présenter une longueur correspondant au diamètre des micro-perforations de la plaque de recouvrement.

Typiquement, les segments ajourés sur la ligne de rupture mesurent de 0,1 à 2 mm, de préférence de 0,3 à 1,8 mm, et de façon particulièrement préférée, de 0,5 à 1,5 mm. Avantageusement, l'espacement entre les segments ajourés sur la ligne de rupture est compris entre 0,1 à 2 mm, de préférence entre 0,3 à 1,8 mm et, de façon particulièrement préférée, entre 0,5 à 1,5 mm.

Maintenant, les segments ajourés peuvent correspondre aux microperforations qui sont alors disposées à intervalles réguliers le long de la ligne de rupture prédéfinie.

De tels segments ajourés assurent en tout cas une rupture maîtrisée sans compromettre la rigidité de la plaque de recouvrement avant son retrait.

Maintenant, la ligne de rupture prédéfinie peut également être formée, au sein de la plaque de recouvrement, d'un matériau de résistance mécanique moindre que le matériau présent de part et d'autre de cette ligne.

On entend par résistance mécanique, la mesure de la capacité d'un matériau à supporter des contraintes sans subir de déformation ou de rupture. Elle est exprimée en termes de contrainte à la rupture, quantifiée en mégapascals (MPa), représentant la force maximale que le matériau peut endurer par unité de surface avant de céder.

Typiquement, pour une plaque de recouvrement en titane, tels que les alliages Ti-6AI-4V ou Ti-CP Grade 2, les matériaux de résistance mécanique significativement réduite peuvent inclure le polylactide (PLA), le polyglycolide (PGA), ou le polycaprolactone (PCL) renforcé, ainsi que l'hydroxyapatite (HA), le phosphate de calcium amorphe, ou encore les alliages de titane-tantale (Ti-Ta) ou titane-magnésium (Ti-Mg).

Alternativement, pour une plaque de recouvrement en titane de grade 2, on pourra utiliser du titane de grade 1 pour former la ou les lignes de rupture prédéfinies au sein de cette plaque de recouvrement.

Avantageusement, le matériau de résistance mécanique moindre présente une contrainte à la rupture (CR2) réduite, par rapport à la contrainte à la rupture du matériau de la plaque de recouvrement (CR1) présent de part et d'autre de la ligne de rupture prédéfinie, et est tel que la valeur du rapport CR2/CR1 est d'au plus 1/2 (soit une contrainte réduite de moitié), de préférence d'au plus 1/3 et, de façon particulièrement préférée, d'au plus 1/4.

Selon un autre mode de réalisation préférée, l'une au moins des extrémités de ladite au moins une ligne de rupture prédéfinie comprend un élargissement de celle-ci. Un tel élargissement correspond au moins à un doublement, voir à un triplement de la largeur de la ligne de rupture prédéfinie.

Typiquement, l'élargissement au niveau de cette extrémité présentera une largeur comprise entre 3 et 10 mm, de préférence entre 4 et 8 mm.

Selon un autre mode de réalisation préféré, l'extrémité de l'au moins une ligne de rupture prédéfinie comprenant un élargissement de celle-ci est l'extrémité non-périphérique de cette au moins une ligne de rupture, laquelle extrémité :
- se positionne, sur la plaque de recouvrement, au niveau d'un futur site d'implantation dans l'os de la mâchoire après régénération osseuse et retrait de la plaque de recouvrement, et
- présente, de préférence, une forme distincte de celle de la ligne de rupture prédéfinie.

A ce titre, la forme et la dimension de l'élargissement à cette extrémité non-périphérique de la ligne de rupture prédéfinie seront choisies de sorte à faciliter son identification (en vue de son ouverture) par le praticien.

Avantageusement, la forme de cette extrémité de la ligne de rupture prédéfinie pourra être de tout type géométrique, par exemple circulaire, hexagonale ou octogonale, de préférence circulaire.

Typiquement, un élargissement prend la forme d'un cercle à cette extrémité de la ligne de rupture prédéfinie.

Avantageusement encore, le fond (de la forme) de cette extrémité ne sera pas plat. De préférence, la forme de cette extrémité sera telle qu'elle présentera un fond arrondi ou conique. Un tel fond arrondi ou conique présente l'avantage de contribuer au guidage/centrage d'un foret, notamment en vue d'une opération de forage en son sein par le praticien.

Comme mentionné précédemment, la surface périphérique de la plaque de recouvrement s'appuyant sur l'os sain, et encadrant ledit défaut osseux, est préférentiellement dépourvue de micro-perforations.

Une telle surface périphérique constituera une bande continue ou discontinue, qui forme tout ou partie du pourtour de la plaque de recouvrement, dont la largeur est comprise entre 0,5 et 5 mm, de préférence entre 1 et 3 mm.

Dans le cas où cette surface périphérique ne comprendra pas de microperforations, elle pourra présenter une épaisseur plus faible que la surface microperforée se positionnant sur le défaut osseux.

Avantageusement, l'extrémité « périphérique » de l'au moins une ligne de rupture, laquelle extrémité est la plus proche du pourtour de la plaque de recouvrement, est positionnée au sein de cette bande ou surface périphérique de la plaque de recouvrement ou à sa proximité immédiate.

Selon un mode de réalisation préféré, l'extrémité « périphérique » de l'au moins une ligne de rupture prédéfinie est positionnée au sein de cette bande ou surface périphérique de la plaque de recouvrement ou à sa proximité immédiate, laquelle surface périphérique constitue en outre une bande discontinue dont la largeur est plus faible, voire nulle, dans le prolongement de cette au moins une ligne de rupture prédéfinie.

De préférence, ce rétrécissement est progressif et est réalisé de sorte à éviter la formation de tout angle vif à la périphérie de la plaque de recouvrement susceptible de blesser le périoste ou les tissus mous.

Selon un mode de réalisation préféré, la surface périphérique comprend au moins un moyen de préhension sur au moins un des 2 côtés encadrant le rétrécissement venant dans le prolongement de ladite au moins une ligne de rupture prédéfinie.

La « zone de préhension » désigne un aménagement structural de la plaque de recouvrement qui est optimisé pour la transmission du couple et la limitation du glissement lors de sa manipulation (mécanique ou manuelle). Typiquement, une zone de préhension peut prendre la forme d'une configuration spécifique de la bordure (languette), d'une proéminence, d'une protubérance, d'une encoche, ou encore d'un méplat antidérapant.

De préférence, ledit au moins un moyen de préhension prend la forme d'un élargissement de la bande discontinue sur au moins un des 2 côtés encadrant ce rétrécissement venant dans le prolongement de ladite au moins une ligne de rupture prédéfinie, lequel élargissement forme une languette.

Selon un mode de réalisation de l'invention, la plaque de recouvrement comprend au moins une zone de préhension prédéfinie, de préférence au niveau de la surface périphérique de la plaque de recouvrement.

Avantageusement, la zone de préhension est une configuration spécifique de la bordure de la plaque de recouvrement selon l'invention.

De préférence la zone de préhension présente une surface texturée telle que des stries de 50 µm à 200 µm, de préférence 70 µm à 150 µm, de façon particulièrement préférée de 100 µm à 130 µm.

En lien maintenant avec le au moins un moyen de fixation, celui-ci peut prendre la forme d'une broche, d'une vis, d'un clou ou d'une colle adaptée à l'os.

Selon un mode de réalisation préféré, ledit au moins un moyen de fixation prend avantageusement la forme d'une vis ou d'un clou.

Typiquement, la plaque de recouvrement comprend alors en outre au moins un orifice adapté pour le passage dudit clou ou de ladite vis de sorte à permettre la fixation, par ledit clou ou ladite vis, de la plaque de recouvrement sur l'os.

Ledit au moins un orifice de passage est positionné, sur la plaque de recouvrement, de sorte à coïncider avec une zone d'os sain entourant le défaut osseux.

Avantageusement, ladite au moins une plaque de recouvrement ne comprend aucun orifice de passage positionné sur la surface de la plaque de recouvrement venant en appui sur le côté interne de l'os (vers l'intérieur de la bouche).

Les exemples qui suivent sont fournis à titre d'illustration et ne sauraient limiter la portée de la présente invention.

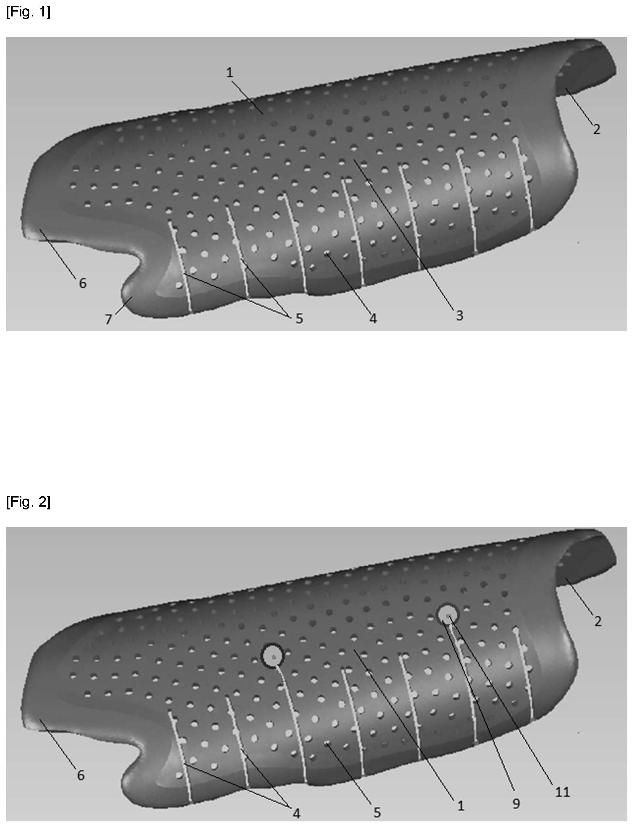

Figures 1 à 4: Représentations de différents modes de réalisation d'une plaque de recouvrement selon l'invention.
- Plaque de recouvrement (1)
- face interne (2)
- face externe (3)
- micro-perforations (4)
- lignes de rupture prédéfinies (5)
- surface périphérique de la plaque (6)
- bord arrondis (7)
- moyens de préhension de type languette (8)
- élargissement non périphérique (9)
- rétrécissement (10) de la surface périphérique (6) dans le prolongement des lignes de rupture prédéfinies (5)
- fond conique (11)
- extrémité périphérique (12) des lignes de rupture prédéfinies Les différentes configurations de plaque de recouvrement (1) présentées dans les figures 1 à 4 sont typiquement fabriquée par impression 3D (fusion laser de poudre de titane). Cette fabrication est rendue possible grâce à une conception sur un système informatique, permettant de calculer et d'adapter précisément la géométrie de la plaque au défaut osseux, notamment au niveau de la mâchoire à laquelle elle est destinée.

Pour ces différentes configurations, on optera pour une plaque de recouvrement (1) en titane de grade 1 avec une épaisseur centrale de 0,7 mm qui permet d'allier rigidité structurale et légèreté tout en épousant la morphologie du défaut osseux à traiter auquel elle est destinée.

Dans ces différentes configurations, la plaque de recouvrement (1) présente une face interne (2), orientée vers le site de régénération (os ou substitut osseux), et une face externe (3) positionnée en regard des tissus mous buccaux pour limiter les frottements et les risques d'ulcération.

Dans les différentes configurations présentées aux figures 1 à 4, cette plaque de recouvrement (1) intègre sept lignes de rupture prédéfinies (5) dont l'épaisseur, entre leurs deux extrémités, est nulle. Ces lignes rupture prédéfinies (5) sont disposées transversalement à l'axe longitudinal de la plaque sur 25 à 35 % de sa longueur, depuis la périphérie (6) de la plaque de recouvrement vers son centre. Les lignes de rupture prédéfinies (5) permettent une ouverture contrôlée de la plaque de recouvrement sous traction manuelle, facilitant ainsi l'accès au site régénéré et le retrait progressif et sans altérer le tissu néoformé de la plaque de recouvrement.

Dans ces différentes configurations, la plaque de recouvrement (1) présente en outre, sur sa surface recouvrant le site de défaut osseux des micro-perforations (4) circulaires. Ces microperforations (4) assurent la diffusion de nutriments et une vascularisation optimale, tout en bloquant la prolifération de tissus mous ou osseux indésirables grâce à un diamètre calibré. A noter que dans ces configurations toujours, les plaques de recouvrement sont dépourvues de micro-perforations (4) sur leur surface périphérique ou bande (6). Cette bande (6) présente une largeur de 1,5 mm le long du pourtour de la plaque de recouvrement et vient en appui direct sur l'os sain péri-lésionnel, assurant ainsi une interface biomécanique homogène. Cette surface périphérique (6) présente en outre des bords arrondis (7) (rayon : 0,2 à 0,5 mm) et épouse l'anatomie de l'os sain. De tel bords arrondis (7) permettent d'éviter de blesser le patient. A noter que la stabilité de la plaque de recouvrement (1) est telle, qu'elle ne nécessite aucune fixation par vis ou clou dans sa surface venant en appui sur le côté interne de l'os (vers l'intérieur de la bouche).

Par rapport au mode de réalisation illustré à la figure 1, la plaque de recouvrement (1) illustrée à la figure 2 présente des lignes de rupture prédéfinies (5) dont l'extrémité non périphérique présente un élargissement circulaire (9) pour un repérage aisé et une ouverture manuelle précise par le praticien. Cet élargissement (9) de forme circulaire présente un fond non plat de type conique (11) qui sert de puit de centrage pour le foret du praticien, guidant et stabilisant l'instrument afin d'effectuer un perçage précis au niveau du futur site implantaire.

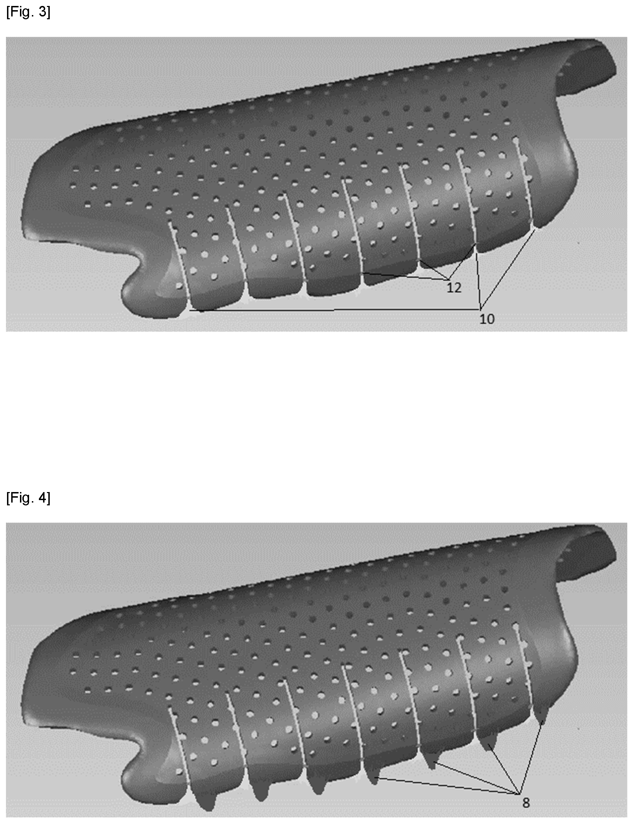

Par rapport au mode de réalisation illustré à la figure 1, la plaque de recouvrement (1) illustrée à la figure 3 présente des lignes de rupture prédéfinies (5) dont l'extrémité périphérique (12) est positionnée au sein de la surface périphérique (6) de la plaque de recouvrement (1), laquelle surface périphérique (6) prend alors la forme d'une bande discontinue avec une largeur plus faible (un amincissement) (10) dans le prolongement de chaque ligne de rupture prédéfinie (5).

Par rapport au mode de réalisation illustré à la figure 3, la plaque de recouvrement (1) illustrée à la figure 4 présente en outre des moyens de préhension (8) à destination du praticien qui se manifeste par une configuration particulière de sa surface périphérique (6) en forme de languettes (8). Cette configuration, permet d'offrir une prise ergonomique et sécurisée, permettant au praticien de manipuler aisément la plaque de recouvrement lors de son retrait, voir même de sa mise en place.

Régénération osseuse d'un défaut osseux de la mâchoire en vue de la pose d'implants dentaires

Dans le cadre d'un défaut osseux au sein de la mâchoire, il est nécessaire de permettre une régénération de ce défaut osseux afin de pouvoir envisager la pose d'implants dentaires.

Pour ce faire, une incision de la gencive est effectuée de sorte à accéder à ce défaut osseux. Cette incision est opérée en opérant une élévation du lambeau de gencive consécutif de l'incision. Le praticien peut alors mettre en œuvre, au niveau du défaut osseux, le matériau destiné à la régénération osseuse.

Une fois ce matériau mis en place, le praticien opère la pose d'une plaque de recouvrement (1) dont la nature (malléable) permet un parfait ajustement sur l'os de la mâchoire du patient. Cette pose est terminée par la fixation, dans l'os sain de la mâchoire entourant le défaut osseux, de deux vis se positionnant dans deux orifices de passage positionnés sur la surface de la plaque de recouvrement (1) venant en appui sur le côté externe de l'os (vers l'extérieur de la bouche).

La fermeture du lambeau, sur la plaque de recouvrement (1), est ensuite être réalisée par le praticien avec une suture (de préférence résorbable).

Après cicatrisation, la régénération osseuse peut s'opérer sous la plaque de recouvrement (1) pendant une période qui peut aller jusqu'à plusieurs mois voire une année.

Au terme de la régénération osseuse, le praticien rouvre le site opératoire par une incision afin d'exposer la plaque de recouvrement (1).

Après cette période de régénération, la plaque de recouvrement (1) peut être retirée sans compromettre la structure et donc la solidité de l'os régénéré. En effet, les micro-perforations (4) de celle-ci empêchent l'adhésion à celle-ci des tissus mous et durs.

La dépose de la plaque de recouvrement (1), par le praticien soit s'effectuer progressivement afin de minimiser tout stress mécanique sur l'os nouvellement formé qui impacterait négativement la pose ultérieure de l'implant dentaire.

Dans le cas d'une plaque de recouvrement (1) telle que présentée à la figure 4, le praticien saisit une bandelette (8) à l'aide d'une pince hémostatique ou d'une pince type Adson. Il exerce alors une traction contrôlée ce qui provoque une ouverture nette au niveau de la ligne de rupture prédéfinie (5) associé et ce jusqu'à ce qu'au point de rupture terminal, à savoir l'extrémité non périphérique de la ligne de rupture prédéfinie (5).

Dans le cas d'une plaque de recouvrement (1) telle que présentée à la figure 2, le praticien effectue un forage au sein de la plaque de recouvrement (1) au niveau de l'élargissement circulaire (9) de forme circulaire de l'extrémité non périphérique d'une ligne de rupture prédéfinie (5). Le fond conique (11) permet un parfait centrage du foret. Une fois le perçage effectué (au niveau du futur site d'implantation), le praticien peut saisir les bords du trou percé à l'aide d'une pince hémostatique ou d'une pince type Adson pour exercer une traction contrôlée provoquant une ouverture nette au niveau de la ligne de rupture prédéfinie (5) associée et ce jusqu'à ce qu'à l'extrémité périphérique de la ligne de rupture prédéfinie (5).

Pour l'une ou l'autre configuration, le praticien réitère cette opération pour plusieurs lignes de rupture prédéfinies jusqu'à permettre une dissociation suffisante de la plaque de recouvrement (1) de la zone régénérée. Le praticien peut alors, avec une traction douce et progressive, décoller la surface de plaque de recouvrement (1) encore associée à l'os régénéré. Pour se faire, u léger mouvement de bascule contrôlé (médial-latéral) peut être effectué afin de minimiser les tensions sur l'os néoformé durant l'extraction finale.

Au final, le retrait de la plaque de recouvrement (1) selon l'invention ne compromet pas la structure et donc la solidité de l'os régénéré. Il est alors possible de poser, dans cet os régénéré, des implants dentaires, notamment au niveau du site de perçage (9) de la configuration présentée en figure 2..]

## Revendications

1. Un dispositif destiné à la régénération osseuse d'un défaut osseux au sein d'un os de la mâchoire qui comprend :
i) au moins une plaque de recouvrement (1) dont :
- la forme permet de recréer la surface de l'os de la mâchoire, préalablement à l'apparition du défaut osseux et en recouvrant ledit défaut osseux, tout en s'appuyant, au moins partiellement, sur l'os sain encadrant ledit défaut osseux,
- la surface, recouvrant ledit défaut osseux, présente des micro-perforations (4), et
ii) au moins un moyen de fixation, et
**caractérisé en ce que** ladite i) au moins une plaque de recouvrement (1) comprend, en outre au moins une ligne de rupture prédéfinie (5) qui :
- s'étend, transversalement à l'axe longitudinal de la plaque de recouvrement (1), de la périphérie (6) de la plaque de recouvrement (1) vers son centre, et
- présente une longueur et une orientation permettant de diriger la rupture de la plaque de recouvrement (1) selon un tracé prédéterminé de sorte à opérer une ouverture contrôlée de cette plaque de recouvrement (1) sur au moins 25% de sa largeur, de préférence sur 25% à 75% de sa largeur
- est disposée, par rapport à l'axe longitudinal de la plaque de recouvrement (1), sur la surface de la plaque de recouvrement venant en appui sur le côté interne de l'os et, de préférence, **en ce que** ladite i) au moins une plaque de recouvrement (1) ne comprend aucun orifice de passage positionné sur la surface de la plaque de recouvrement (1) venant en appui sur le côté interne de l'os .

2. Le dispositif selon la revendication 1, **caractérisé en ce que** l'une au moins des extrémités de ladite au moins une ligne de rupture prédéfinie (5) comprend un élargissement de celle-ci, lequel élargissement prend de préférence la forme d'un cercle.

3. Le dispositif selon la revendication précédente, **caractérisé en ce que** l'extrémité de l'au moins une ligne de rupture prédéfinie (5) comprenant un élargissement (9) est l'extrémité non-périphérique de cette au moins une ligne de rupture prédéfinie (5), laquelle extrémité :
- se positionne, sur la plaque de recouvrement (1), au niveau d'un futur site d'implantation dans l'os de la mâchoire après régénération osseuse et retrait de la plaque de recouvrement (1), et
- présente, de préférence, une forme distincte de celle de la ligne de rupture prédéfinie.

4. Le dispositif selon la revendication précédente, **caractérisé en ce que** la forme de cette extrémité est telle qu'elle présente un fond arrondi ou conique (11).

5. Le dispositif selon l'une quelconque des revendications précédentes, **caractérisé en ce que** l'extrémité « périphérique » (12) de l'au moins une ligne de rupture prédéfinie (5) est positionnée au sein de la surface périphérique (6) de la plaque de recouvrement ou à sa proximité immédiate, laquelle surface périphérique (6) constitue en outre une bande discontinue dont la largeur est plus faible, voire nulle, dans le prolongement (12) de cette au moins une ligne de rupture prédéfinie (5).

6. Le dispositif selon la revendication précédente, **caractérisé en ce que** la surface périphérique (6) comprend au moins un moyen de préhension (8) sur au moins un des 2 côtés encadrant son rétrécissement dans le prolongement (12) de ladite au moins une ligne de rupture prédéfinie, lequel moyen de préhension prend de préférence la forme d'un élargissement de la bande discontinue prenant typiquement la forme une languette (8).

7. Le dispositif selon la revendication précédente, **caractérisé en ce que** l'au moins une ligne de rupture prédéfinie (5) est une ligne de moindre épaisseur dont la moindre épaisseur se matérialise sur l'une et/ou l'autre des deux faces de la plaque de recouvrement.

8. Le dispositif selon l'une quelconque des revendications précédentes, **caractérisé en ce que** l'au moins une ligne de rupture prédéfinie (5) est formée, au sein de la plaque de recouvrement (1), d'un matériau de résistance mécanique moindre que le matériau présent de part et d'autre de cette ligne de rupture prédéfinie (5).

9. Une plaque de recouvrement selon la revendication 1, destinée à la régénération osseuse d'un défaut osseux au sein d'un os de la mâchoire, dont :
- la forme permet de recréer la surface de l'os de la mâchoire, préalablement à l'apparition du défaut osseux et en recouvrant ledit défaut osseux, tout en s'appuyant, au moins partiellement, sur l'os sain encadrant ledit défaut osseux,
- la surface, recouvrant ledit défaut osseux, consiste en une surface micro-perforée, présente des micro-perforations (4),
**caractérisée en ce qu'**elle comprend :
- au moins une ligne de rupture prédéfinie (5) qui :
- s'étend, transversalement à l'axe longitudinal de la plaque de recouvrement (1), de la périphérie (6) de la plaque de recouvrement (1) vers son centre, et
- présente une longueur et une orientation permettant de diriger la rupture de la plaque de recouvrement (1) selon un tracé prédéterminé de sorte à opérer une ouverture contrôlée de cette plaque de recouvrement (1) sur au moins 25% de sa largeur, de préférence sur 25% à 75% de sa largeur.
